# EUROPÄISCHE PATENTSCHRIFT

(11) **EP 0 659 734 B1**
(45) Veröffentlichungstag und Bekanntmachung des Hinweises auf die Patenterteilung: **19.03.1997**
(21) Anmeldenummer: 94118078.8
(22) Anmeldetag: 17.11.1994
(51) Int. Cl.: C07C 209/48, C07C 209/26

(54) **Verfahren zur Herstellung von 3-Aminomethyl-3,5,5-trimethylcyclohexylamin**
Process for the preparation of 3-aminomethyl-3,5,5-trimethylcyclohexylamine
Procédé pour la préparation de 3-amino-3,5,5-triméthylcyclohexylamine

(30) Priorität: 22.12.1993 DE 4343890
(43) Veröffentlichungstag der Anmeldung: 28.06.1995
(73) Patentinhaber: Degussa Aktiengesellschaft, 60311 Frankfurt (DE)
(72) Erfinder: Haas, Dr. Thomas, D-60316 Frankfurt (DE); Arntz, Dr. Dietrich, D-61440 Oberursel (DE); Most, Dr. Dieter, D-63486 Bruchköbel (DE)

(56) Entgegenhaltungen:
- EP-A- 0 042 119
- EP-A- 0 449 089
- EP-A- 0 503 246

## Beschreibung

Die Erfindung richtet sich auf ein verbessertes Verfahren zur Herstellung von 3-Aminomethyl-3,5,5-trimethylcyclohexylamin, nachfolgend auch Isophorondiamin oder abgekürzt IPDA genannt, aus 3-Cyano-3,5,5-trimethylcyclohexanon, nachfolgend auch Isophoronnitril oder abgekürzt IPN genannt, durch aminierende Hydrierung mit Wasserstoff und Ammoniak in Gegenwart eines Festbettkatalysators. Das erfindungsgemäße Verfahren erlaubt die kontinuierliche Herstellung von Isophorondiamin in hoher Ausbeute und hoher Reinheit.

Isophorondiamin wird als Ausgangsprodukt zur Herstellung von Isophorondiisocyanat, einer Isocyanatkomponente für Polyurethansysteme, als Aminkomponente für Polyamide und als Härter für Epoxidharze verwendet. Isophorondiamin wird üblicherweise aus Isophoronnitril hergestellt, wobei in Gegenwart von Ammoniak, Wasserstoff und üblichen Hydrierkatalysatoren die Carbonylgruppe in eine Aminogruppe und die Nitrilgruppe in eine Aminomethylgruppe überführt werden. Das Ausgangsprodukt Isophoronnitril läßt sich in bekannter Weise durch Anlagerung von Cyanwasserstoff an Isophoron erhalten - siehe DE-OS 39 42 371.

Gemäß dem in der US 3,352,913 beschriebenen Verfahren zur Herstellung von Isophorondiamin aus Isophoronnitril erfolgt die Hydrierung in Gegenwart von Ammoniak und in Gegenwart von an sich bekannten kobalt-, nickel-, eisen- oder edelmetallhaltigen Katalysatoren bei 50 bis 150 °C und einem Druck von wenigstens 50 bar. Beispielsgemäß erfolgt die Hydrierung in Gegenwart von Methanol als Lösungsmittel unter Verwendung von Suspensions- oder Festbettkatalysatoren. Außer dem gewünschten Isophorondiamin entstehen in größerer Menge Nebenprodukte, wie insbesondere 3-Aminomethyl-3,5,5-trimethylcyclohexanol (Isophoronaminoalkohol, IPAA). Als nachteilig an diesem Verfahren erweisen sich die geringe Ausbeute sowie ein erheblicher Anteil an Nebenprodukten.

Im Bestreben, eine höhere Ausbeute an IPDA zu erhalten und den Zwangsanfall an IPAA zu minimieren, lehrt die DE-OS 30 11 656 ein zweistufiges Verfahren, wobei in der ersten Stufe IPN katalysatorfrei mit überschüssigem Ammoniak in 3-Cyano-3,5,5-trimethyl-iminocyclohexan überführt und dieses in der zweiten Stufe zu IPDA hydriert wird. Nachteilig an diesem Verfahren ist, daß außer dem eigentlichen Hydrierreaktor ein spezieller Iminbildungsreaktor erforderlich ist.

Gemäß der EP-B 0 042 119 wird eine weitere Verbesserung des Verfahrens darin gesehen, das Isophoronnitril vor der Reaktion desselben mit Ammoniak und Wasserstoff in Gegenwart von Hydrierkatalysatoren bei Temperaturen von 10 bis 120 °C und Drucken von 1 bis 300 bar einer Vorreaktion mit Ammoniak in Gegenwart von anorganischen und organischen Ionenaustauschern in der Ammoniumform als Iminbildungskatalysatoren zu unterwerfen. Während das Volumenverhältnis Isophoronnitril zu Ammoniak in der Iminbildungsstufe 1 zu 0,5 bis 20 betragen soll, wird dieses Verhältnis in der Hydrierstufe auf 1 zu 10 bis 20 erhöht. Das Verfahren, das sich in Rieselbettreaktoren ausführen läßt, führt zwar zu einer hohen Ausbeute an IPDA sowie zu einer hohen Reinheit, die Wirtschaftlichkeit des Verfahrens wird aber durch die Verwendung von zwei unterschiedlichen Katalysatoren sowie den hohen Ammoniaküberschuß, der einen sehr hohen Druck und damit eine aufwendige Hydrierapparatur erforderlich macht, beeinträchtigt.

In der EP-B 0 042 119 wird auch ein Vergleichsbeispiel offenbart, wobei Isophoronnitril und flüssiger Ammoniak in einen mit handelsüblichem Kobaltkatalysator beschickten Hydrierreaktor von oben hineingepumpt werden. Das Reaktionssystem wird mit H₂ auf 270 bar gehalten; ein gewisser Gasstrom wird eingestellt und eine gewisse Menge Abgas ausgeschleust. Das den Reaktor unten verlassende Reaktionsgemisch wird der destillativen Aufarbeitung zugeführt. Bei dieser Ausführungsform werden trotz etwa quantitativer Umsetzung des Isophoronnitrils nur 48 % Isophorondiamin und daneben viele Nebenprodukte erhalten. Eine Anregung, anstelle eines Gemischs aus Isophoronnitril und Ammoniak ein Isophoron, Ammoniak und organisches Lösungsmittel enthaltendes Gemisch bei wesentlich geringem Druck und ohne das Erfordernis einer vorgeschalteten Iminbildungsreaktion über einen Rieselbettreaktor zu leiten, kann diesem Dokument nicht entnommen werden.

Aus der EP-A 0 449 089 ist ein weiteres Verfahren zur Herstellung von Isophorondiamin bekannt: In zwei räumlich voneinander getrennten Reaktionsräumen wird eine Lösung von Isophoronnitril in Tetrahydrofuran zunächst mit überschüssigem Ammoniak an aciden Metallkatalysatoren umgesetzt und das Reaktionsgemisch in einem zweiten Reaktionsraum mit Wasserstoff in Gegenwart von überschüssigem Ammoniak an kobalt-, nickel-, ruthenium- und/oder anderen edelmetallhaltigen Katalysatoren und gegebenenfalls basischen Komponenten bei hohem Druck hydriert. Beispielsgemäß wird das die erste Reaktionsstufe verlassende Gemisch von unten nach oben durch den Hydrierreaktor gefahren; der Reaktor wird als Blasenreaktor betrieben. Hinweise, den Reaktor auch als Rieselbettreaktor zu verwenden und diesem unmittelbar ein Gemisch aus Isophoronnitril, Ammoniak und einem organischen Lösungsmittel zuzuführen, lassen sich aus diesem Dokument nicht herleiten.

Die EP-A 0 394 967 lehrt ein Verfahren zur Aminierung von Carbonylnitrilen und Iminonitrilen und umfaßt auch die Herstellung von Isophorondiamin aus Isophoronnitril. Das Ausgangsprodukt wird unter Bedingungen der reduktiven Aminierung, also in Gegenwart von Wasserstoff, Ammoniak und einem Hydrierkatalysator bei mäßigen Temperaturen zunächst in das Aminonitril überführt; anschließend wird die Nitrilgruppe in Gegenwart eines gegenüber Nitrilgruppen hydrierwirksamen Hydrierkatalysators bei höhrerer Temperatur in eine Aminomethylgruppe überführt. Das Verfahren kann sowohl in Reaktoren unter Verwendung von Suspensionskatalysatoren als auch in Reaktoren unter Verwendung von Festbettkatalysatoren durchgeführt werden. Obgleich dieses Verfahren bei niedrigen Drucken durchgeführt werden kann, wird als wesentlicher Nachteil angesehen, daß ein streng einzuhaltendes Temperaturprogramm während der beiden Reaktionsstufen gefahren werden muß, wodurch die Raum-Zeit-Ausbeute und damit die Wirtschaftlichkeit des Verfahrens erheblich sinken. Zusätzlich entspricht die Produktqualität, sofern nicht spezielle Promotoren zusätzlich zum Einsatz kommen, wegen des meist zu hohen Gehalts an destillativ nicht abtrennbarem 3-Cyano-3,5,5-trimethyl-aminocyclohexan nicht den Anforderungen.

Eine Reduzierung des Drucks bei der Herstellung von Isophorondiamin durch aminierende katalytische Hydrierung von Isophoronnitril ist gemäß JP-A-4-300852 auch dadurch möglich, daß in Gegenwart eines Trägerkatalysators mit Ruthenium hydriert wird. Dieses Dokument betrifft aber nur Bedindungen für die Suspensionshydrierung nicht aber jene unter Verwendung eines Festbettreaktors.

Eine Übersicht über ein technisch genutztes Verfahren zur Herstellung von Isophorondiamin aus Isophoronnitril ist aus SRI International Report Nr. 1D "Isocyanates" by YU-REN CHIN (Juli 1983) bekannt geworden: In dem hier beschriebenen Verfahren wird ein Gemisch aus Isophoronnitril, Methanol und Ammoniak mit Wasserstoff über einen Festbettreaktor mit einem Trägerkatalysator - Kobalt auf Kieselgur - geschickt. Das Katalysatorbett ist bei der beschriebenen Ausführungsform stets geflutet (Blasensäule). Als Betriebsdruck werden 150 bar angegeben. Ferner wird Wasserstoff im Überschuß eingesetzt, und dieser Überschuß muß nach der Reaktion vom Reaktionsgemisch getrennt, gereinigt und anschließend nach Kompression zurückgeführt werden. Sowohl der hohe Betriebsdruck als auch der technische Aufwand zur Rückführung des Wasserstoffs mindern die Wirtschaftlichkeit dieses Verfahrens. Wie die Erfinder der vorliegenden Erfindung feststellten - siehe nicht erfindungsgemäßes Beispiel 3b - kommt es bei dieser Ausführungsform zu einer vermehrten Bildung von schwer abtrennbaren Nebenprodukten und Minderung der Ausbeute.

Die Aufgabe der vorliegenden Erfindung besteht somit darin, die Nachteile der vorbekannten Verfahren zur Herstellung von Isophorondiamin aus Isophoronnitril unter Verwendung eines mit einem Festbettkatalysator beschickten Reaktors dahingehend zu verbessern, daß die Wirtschaftlichkeit des Verfahrens erhöht wird. Insbesondere sollte der Aufwand für die technische Einrichtung gegenüber den vorbekannten Verfahren vermindert werden. Isophorondiamin sollte zudem in hoher Ausbeute erhalten und mittels üblicher destillativer Maßnahmen in sehr reiner Form aus dem Reaktionsgemisch isoliert werden können.

Gefunden wurde ein Verfahren zur Herstellung von 3-Aminomethyl-3,5,5-trimethylcyclohexylamin (Isophorondiamin, IPDA) aus 3-Cyano-3,5,5-trimethylcyclohexanon (Isophoronnitril, IPN), indem ein Gemisch aus Isophoronnitril, einem organischen Lösungsmittel aus der Reihe der C₁- bis C₃-Alkohole und Ammoniak bei einem Druck im Bereich von 3 bis 10 MPa und einer Temperatur im Bereich von 80 bis 150 °C in Gegenwart mindestens eines Hydrierkatalysators aus der Reihe der Kobalt- und/oder Ruthenium-Festbettkatalysatoren mit Wasserstoff hydriert und das Reaktionsgemisch destillativ aufgearbeitet wird, das dadurch gekennzeichnet ist, daß man die aminierende Hydrierung in einem Rieselbettreaktor durchführt, wobei das Gemisch aus Isophoronnitril, organischem Lösungsmittel und Ammoniak am oberen Ende des Reaktors auf das Katalysatorbett aufgegeben und das nach Durchrieseln des Katalysatorbetts erhaltene Reaktionsgemisch am unteren Ende des Reaktors abgenommen wird.

Die erfindungsgemäße Umsetzung wird in einem üblichen Rieselbettreaktor durchgeführt. Die Bauarten derartiger Reaktoren sind dem Fachmann bekannt: In einem Behälter ist der Festbettkatalysator in Form einer oder mehrerer Schichten angeordnet; der Reaktor verfügt ferner über Vorrichtungen zum Temperieren der Katalysatorschichten, um die Aufrechterhaltung der gewünschten Temperatur in der jeweiligen Katalysatorschicht zu gewährleisten. Anstelle eines einzigen Rieselbettreaktors können auch mehrere Rieselbettreaktoren hintereinander geschaltet werden, wobei das aus dem ersten Reaktor austretende Reaktionsgemisch am Kopf des zweiten Reaktors wieder aufgegeben wird. Der bzw. die Rieselbettreaktoren verfügen ferner über geeignete Vorrichtungen zum Aufgeben der Reaktionspartner, hier also des Isophoronnitril, Ammoniak und Lösungsmittel enthaltenden Gemischs sowie des Wasserstoffs, ferner Vorrichtungen zum Verteilen der Flüssigkeit auf der Oberfläche des ersten Katalysatorbetts und schließlich geeignete Austragsvorrichtungen für das den Reaktor verlassende Reaktionsgemisch.

Es ist ein wesentliches Kennzeichen des erfindungsgemäßen Verfahrens, daß das zu hydrierende Reaktionsgemisch über das Katalysatorbett rieselt und innerhalb des Katalysatorbetts auch ein ausreichend großes Gasvolumen existiert, um den Wasserstofftransport durch den Flüssigkeitsfilm an die Katalysatoroberfläche zu ermöglichen. Es wurde gefunden, daß die Rieselbettfahrweise maßgeblich zum Erhalt einer hohen Produktausbeute und vor allem einer hohen Selektivität verantwortlich ist. Sofern der Reaktor nicht als Rieselbettreaktor, sondern als Blasenreaktor betrieben wird, wobei also das Katalysatorbett stets geflutet ist, wird unter sonst gleichen Reaktionsbedingungen bezüglich Temperatur, Druck und Konzentration der Reaktionspartner eine wesentlich geringere Ausbeute an Isophorondiamin erhalten; die Blasenfahrweise führt auch zu einem anderen Nebenproduktspektrum, wobei insbesondere Methyl-Isophorondiamin (Methyl-3-Aminomethyl-3,5,5-trimethylcyclohexyl-amin) gebildet wird. Das genannte Methyl-Isophorondiamin läßt sich unter üblichen Bedingungen nicht destillativ von Isophorondiamin abtrennen, so daß die Bildung des genannten Nebenprodukts bereits bei der Herstellung des Isophorondiamin-Rohproduktes vermieden werden muß. Überraschenderweise bildet sich unter den Bedingungen des erfindungsgemäßen Verfahrens das genannte Nebenprodukt nur in sehr geringer Menge. Ein weiterer Nachteil der Blasenfahrweise ist, daß Wasserstoff im Überschuß zugesetzt werden muß, um eine H₂-Sättigung der Lösung zu erhalten, was eine aufwendige Rezyklierung des H₂-Überschusses zur Folge hat.

Bei der Durchführung des erfindungsgemäßen Verfahrens kann Wasserstoff dem Reaktor entweder im Überschuß zugeführt werden oder in einer solchen Menge, daß kein Wasserstoff aus dem Reaktor ausgetragen und rezykliert werden muß. Die Zufuhr des Wasserstoffs im Überschuß wird weniger bevorzugt, weil der technische Aufwand zur Abtrennung des H₂-Überschusses, zur Kondensation von im ausgetragenen Wasserstoff enthaltenem Ammoniak und organischen Lösungsmittel sowie zur Kompression des gereinigten Wasserstoffs und dessen Rezyklierung erheblich ist und wegen der dadurch verursachten höheren Investition die Wirtschaftlichkeit des Verfahrens mindert; zudem wurde gegenüber der Fahrweise ohne H₂-Überschuß sogar eine geringere Ausbeute an IPDA festgestellt. Vorzugsweise wird somit Wasserstoff nicht im Überschuß zugeführt, sondern nur in einer solchen Menge, welche zur Aufrechterhaltung des gewünschten Betriebsdrucks erforderlich ist.

Die aminierende Hydrierung wird bei einem Druck im Bereich von 3 bis 10 MPa, vorzugsweise bei 5 bis 8 MPa, durchgeführt. Durch die genannten mäßigen Betriebsdrucke, welche bei Verwendung der anspruchsgemäßen Gemische aus Isophoronnitril, Ammoniak und Lösungsmittel und der Rieselbettfahrweise unter den anspruchsgemäßen Temperaturbedingungen möglich sind, wird die Wirtschaftlichkeit gegenüber Verfahren, welche einen hohen Betriebsdruck erfordern, erhöht, indem die Apparatur ein geringeres Investitionsvolumen erfordert.

Das erforderliche Volumen an Festbettkatalysator richtet sich nach dem vom Betriebsdruck, der Temperatur und Katalysatoraktivität abhängigen LHSV-Wert (liquid hourly space velocity), der eingehalten werden muß, um einen quantitativen Umsatz an Isophoronnitril zu erzielen. Üblicherweise liegt der LHSV-Wert bei oder besser über 0,5 h⁻¹. Vorzugsweise liegt der LHSV-Wert zwischen 0,8 und 1,5 h⁻¹. Ein LHSV-Wert im letztgenannten Bereich ist besonders gut zu erzielen, wenn die Umsetzung bei einer Temperatur im Bereich zwischen 90 und 130 °C, bei einem Druck zwischen 5 und 8 MPa durchgeführt wird.

Zusätzlich zu den vorgenannten Parametern Druck, Temperatur und Katalysatoraktivität wird der LHSV-Wert auch von den gewählten Konzentrationsverhältnissen an Isophoronnitril, Ammoniak und Lösungsmittel sowie der Art des eingesetzten Lösungsmittels beeinflußt. Zweckmäßigerweise enthält das auf das Katalysatorbett aufgegebene Gemisch 10 bis 40 Gew.-% und vorzugsweise 10 bis 30 Gew.-% Isophoronnitril und 10 bis 40 Gew.-%, vorzugsweise 20 bis 40 Gew.-% Ammoniak. Als Lösungsmittel können Methanol, Ethanol, n-Propanol und iso-Propanol verwendet werden. Bevorzugt wird Methanol als Lösungsmittel. Isophoronnitril, Ammoniak und das Lösungsmittel werden in einem solchen Verhältnis gemischt, daß eine im wesentlichen homogene Lösung resultiert. Im Prinzip können die zuvor genannten Grenzwerte für Ammoniak und Isophoronnitril auch unter- oder überschritten werden, sofern hierbei eine homogene Lösung resultiert. Sofern ein weniger als 10 Gew.-% Isophoronnitril enthaltendes Gemisch eingesetzt wird, wirkt sich dies negativ auf die Wirtschaftlichkeit des Verfahrens aus.

Außer den vorgenannten Bestandteilen kann das auf den Katalysator aufzugebende Gemisch höher oder tiefer als Isophorondiamin siedende Fraktionen aus der destillativen Aufarbeitung des aus dem Rieselbettreaktor abgezogenen Reaktionsgemischs enthalten. Derartige Fraktionen können außer Resten an Isophorondiamin auch solche Nebenprodukte enthalten, aus welchen sich unter den Reaktionsbedingungen erneut Isophorondiamin bildet. Durch Rückführung derartiger Fraktionen in das einzusetzende Gemisch läßt sich die Ausbeute an Isophorondiamin deutlich steigern. Besonders vorteilhaft ist es, die nach dem Isophorondiamin siedende Fraktion, welche außer Resten an Isophorondiamin 3,3,5-Trimethyl-6-imino-7-aza-bicyclo-[3,2-1]-octan als Hauptprodukt enthält, zusammen mit dem Gemisch aus Isophoronnitril, Ammoniak und Lösungsmittel dem Rieselbettreaktor zuzuführen. Durch die Rückführung der das vorgenannte Nebenprodukt - eine bicyclische Verbindung mit Amidinstruktur - enthaltenden Fraktion ist es möglich, die Ausbeute an Isophorondiamin nennenswert zu steigern und damit die Wirtschaftlichkeit des Verfahrens zu erhöhen.

Bei den erfindungsgemäß zu verwendenden Katalysatoren handelt es sich um Kobalt- und/oder Ruthenium-Festbettkatalysatoren. Diese Katalysatoren liegen üblicherweise als Pellets, Strangpreßlinge oder andere Formlinge vor. Besonders zweckmäßig ist die Verwendung von Trägerkatalysatoren, wobei sich Kobalt oder Ruthenium auf einem Träger mit ausreichend großer spezifischer Oberfläche befinden. Üblicherweise liegt die spezifische Oberfläche der erfindungsgemäß einsetzbaren Trägerkatalysatoren im Bereich zwischen 5 und 500 qm/g (gemessen nach BET mit Stickstoff) .

Der Kobaltgehalt der Trägerkatalysatoren liegt im allgemeinen zwischen 10 und 70 Gew.-% und vorzugsweise zwischen 30 und 50 Gew.-%, bezogen auf den Trägerkatalysator. Die Oberfläche des Trägers, bei welchem es sich vielfach um oxidische oder silikatische Materialien handelt, ist teilweise oder im wesentlichen vollständig mit Kobalt bedeckt, wobei unterhalb der Oberfläche herstellungsbedingt auch Kobaltoxid anwesend sein kann. Als Trägermaterial kommen beispielsweise Oxide aus der Reihe Siliciumdioxid, Aluminiumoxid und Titandioxid, ferner Silikate, wie Aluminium- und Calciumsilikat sowie natürlich vorkommende und synthetisch hergestellte Zeolithe und Glasfritten infrage. Obwohl handelsübliche Trägerkatalysatoren mit hohem Co-Gehalt, wie Katalysatoren mit 50 Gew.-% Co auf Aluminiumsilikat, als im wesentlichen vollständig mit Co bedeckt beschrieben werden, wurde gefunden, daß Katalysatoren mit unterschiedlichem Träger aber gleicher spezifischer Oberfläche derselben und gleichem Co-Gehalt die Selektivität und das Nebenproduktspektrum beeinflussen. Offensichtlich ist die Oberfläche des Trägers des Katalysators wenigstens teilweise zugänglich und greift in den katalytischen Prozess ein.

Trägerkatalysatoren mit Ruthenium als katalytisch wirksamer Komponente enthalten aus Kostengründen im allgemeinen nur einen geringen Gewichtsanteil an Ruthenium, üblicherweise zwischen 0,5 und 10 Gew.-%, vorzugsweise zwischen 2 und 5 Gew.-%. Die Herstellung der vorerwähnten Co- und Ru-Katalysatoren, welche üblicherweise einen Imprägnierungs-, Trocknungs- und Formierungsschritt umfaßt, ist der Fachwelt bekannt.

Es wurde festgestellt, daß bei Verwendung eines Kobalt-Trägerkatalysators Isophorondiamin nach dem erfindungsgemäßen Verfahren in höherer Ausbeute erhältlich ist als im Falle der Verwendung eines Ruthenium-Trägerkatalysators. Dies ist insofern überraschend, weil in den vorerwähnten Dokumenten JP-A 4-300852 sowie EP-A 0 394 967 Rutheniumkatalysatoren als besonders wirksam bezüglich der Hydrierung der gegenüber der Carbonylgruppe schwerer zu hydierenden Nitrilgruppe bezeichnet wurden.

Bekanntlich kommt Isophorondiamin in Form von cis- und trans-Isomeren vor - siehe Die Angewandte Makromolekulare Chemie 153 (1987) 1-13, Nr. 2502. Bei der Untersuchung des Isomerenverhältnisses des erfindungsgemäß hergestellten Isophorondiamins zeigte sich, daß bei Verwendung eines Rutheniumkatalysators ganz überwiegend das cis-Isomere gebildet wird: cis-/trans-Isomerenverhältnis gemäß Beispiel 9: 84 zu 16. Unter sonst gleichen Bedingungen, jedoch unter Verwendung eines Kobalt-Trägerkatalysators werden das cis- und trans-Isomere in ähnlicher Menge gebildet: cis-/trans-Isomerenverhältnis gemäß Beispiel 8: 60 zu 40.

Es wurde ferner gefunden, daß es möglich ist, Isophorondiamin mit einem cis-/trans-Isomerenverhältnis oberhalb desjenigen, das unter Verwendung eines Kobalt-Trägerkatalysators erhältlich ist, in hoher Ausbeute herzustellen, wenn man die aminierende Hydrierung in einem Rieselbettreaktor durchführt, in welchem eine obere Schicht aus einem Ruthenium-Trägerkatalysator und eine untere Schicht aus einem Kobalt-Festbettkatalysator angeordnet ist. Besonders geeignete Katalysatoren sind Ruthenium auf γ-Aluminiumoxid und ein Kobalt-Trägerkatalysator mit einem oxidischen oder silikatischen Träger mit einem Kobaltgehalt von 10 bis 70 Gew.-%, bezogen auf den Katalysator. Anstelle eines Reaktors mit zwei darin angeordneten Schichten verschiedener Festbettkatalysatoren können auch zwei Reaktoren hintereinander geschaltet werden, welche jeweils nur einen Festbettkatalysator enthalten; in diesem Fall enthält der erste Reaktor den Ruthenium-Festbettkatalysator und der zweite Reaktor den Kobalt-Festbettkatalysator. Um eine hohe Ausbeute an Isophorondiamin mit dem gewünschten Isomerenverhältnis zu erhalten, ist es vorteilhaft, wenn der an erster Stelle positionierte Ruthenium-Festbettkatalysator 3 bis 30 %, vorzugsweise 5 bis 20 %, des gesamten Katalysatorvolumens ausmacht.

Wie vorstehend ausgeführt wurde, unterscheidet sich das erfindungsgemäße Verfahren in wesentlichen Punkten von gattungsgemäßen vorbekannten Verfahren, woraus wesentliche Vorteile resultieren. Durch die Kombination der Verwendung eines Ausgangsgemischs aus Isophoronnitril, Ammoniak und einem alkoholischen Lösungsmittel und die Rieselbettfahrweise des mit einem Festbett ausgestatteten Reaktors ist es möglich, sowohl eine hohe Produktausbeute an Isophorondiamin sowie eine hohe Selektivität zu erzielen. Die Bildung unerwünschter Nebenprodukte, wie des Methyl-Isophorondiamins, das wegen seiner schweren Abtrennbarkeit die Produktqualität negativ beeinflußt, wird durch die Reaktionsführung weitgehend vermieden. Die zuvor genannte Kombination erfindungswesentlicher Merkmale führt auch dazu, daß die aminierende Hydrierung bei mäßigen Drucken durchführbar ist, so daß der dadurch bedingte Aufwand für die Druckapparatur viel niedriger ist als im Falle einer Apparatur für hohe Drucke. Da es entgegen vorbekannten Verfahren unter Verwendung eines Reaktors mit einem Festbettkatalysator erfindungsgemäß nicht erforderlich ist, Wasserstoff im Überschuß einzusetzen und damit den Überschuß reinigen und rezyklieren zu müssen, entfallen aufwendige Vorrichtungen zur Abtrennung, Reinigung und Rezyklierung des H₂-Überschusses. Ein weiterer wesentlicher Vorteil des erfindungsgemäßen Verfahrens besteht in der unerwartet hohen Standzeit der Katalysatoren, welche auf die Rieselbettfahrweise zurückgeführt wird: Auch nach mehrmonatigem Betrieb wurden weder eine Ausbeuteminderung noch eine Änderung des Nebenproduktspektrums noch eine Änderung des Isomerenverhältnisses festgestellt. Ein weiterer Vorteil besteht darin, Isophorondiamin mit einem bestimmten Isomerenverhältnis innerhalb gewisser Grenzen in hoher Ausbeute gewinnen zu können. Im erfindungsgemäßen Verfahren erübrigt sich auch eine Vorreaktion zwischen dem Isophoronnitril und Ammoniak zwecks Bildung des Isophoronimins, welche in etlichen Dokumenten als wesentlich zum Erhalt einer hohen Ausbeute und hohen Selektivität bei der aminierenden Hydrierung angesehen wurde; erfindungsgemäß wird demgegenüber eine Lösung des Isophoronnitrils in dem verwendeten Lösungsmittel unmittelbar vor dem Rieselbettreaktor mit Ammoniak gemischt und das Gemisch dem Reaktor zugeführt.

Anhand der nachfolgenden Beispiele wird das erfindungsgemäße Verfahren weiter erläutert ohne dieses zu begrenzen.

### Beispiel 1

Ein Reaktionsrohr wird mit 200 ml Hydrierkatalysator gefüllt. Die Einsatzlösung, die IPN und Methanol enthält, sowie flüssiges NH₃, wird oben in den Reaktor gepumpt. Auch der Wasserstoff strömt von oben in das Rohr. Die Reaktionstemperatur wird durch eine Ölheizung auf 120 °C gehalten. Der Druck wird auf 60 bar geregelt. Die Flüssigkeit wird in einem Abscheidegefäß aufgefangen. Der Gasstrom am Reaktoreingang wird so eingestellt, daß sich nach dem Abscheidegefäß ein Gasstrom von 100 Nl/h einstellt.

Als Katalysator wurde ein handelsüblicher Co-Vollkontakt (ca. 50 % Co auf silikatischem Träger) in Form von Strangpreßlingen mit 4-5 mm Durchmesser und Länge eingesetzt. In der Einsatzlösung waren 24 Gew.-% IPN und 76 Gew.-% Methanol enthalten. Davon wurden 130 ml/h und 70 ml/h NH₃ unmittelbar vor der Aufgabe auf den Reaktor gemischt und das Gemisch in den Reaktor gepumpt; der LHSV-Wert betrug also 1 h⁻¹. Nach der Analyse des Produktgemisches ergab sich eine Ausbeute von 88,7 % IPDA, bezogen auf eingesetztes IPN. Außerdem waren 5 % 2-Aza-4,6,6-trimethylbicyclo-[3,2,1]-octan (=Bicyclus) und 4,3 % 3,3,5-Trimethyl-6-imino-7-aza-bicyclo-[3,2,1]-octan (=Amidin) im Produktgemisch enthalten.

### Beispiel 2

Der Versuch wurde in der gleichen Versuchsanlage, wie schon im Beispiel 1 beschrieben, durchgeführt mit dem Unterschied, daß aus dem Abscheidegefäß kein Gasstrom ausgeschleust wurde, so daß nur der verbrauchte Wasserstoff nachdosiert wurde, um den Reaktionsdruck von 60 bar zu halten. Katalysator, Flüssigkeitsströme und Flüssigkeitszusammensetzung waren die gleichen wie im Beispiel 1 beschrieben.

Die Analyse des Produktgemisches dieses Versuches ergab eine IPDA-Ausbeute von 89,2 %, bezogen auf eingesetztes IPN. Außerdem waren 4,5 % Bicyclus und 4,4 % Amidin im Produktgemisch enthalten. Überraschenderweise werden bei der Fahrweise ohne H₂-Überschuß sogar eine höhere Ausbeute an IPDA und eine Reduzierung des Nebenproduktes "Bicyclus" ermöglicht. Dieser Versuch wurde über 2000 h betrieben, ohne daß ein Ausbeuteverlust oder verändertes Produktspektrum zu beobachten waren.

### Beispiele 3a und 3b

Es wurde die gleiche Versuchsanlage wie in Versuch 1 verwendet. Der Hydrierdruck betrug 60 bar, die Reaktionstemperatur 120 °C. Der H₂-Strom wurde so eingestellt, daß aus dem Abscheidegefäß 100 Nl/h H₂ ausgeschleust wurden. In der Einsatzlösung waren 30 Gew.-% IPN und 70 Gew.-% Methanol enthalten. Davon wurden 130 ml/h und zusätzlich 50 ml/h flüssiges NH₃ in den Reaktor gepumpt. Als Katalysator wurden 200 ml Kobaltkontakt eingefüllt. Es wurden zwei Fahrweisen untersucht. In der Rieselbettfahrweise (3a) durchströmten Gas und Flüssigkeit den Reaktor von oben nach unten, in der Blasensäulenfahrweise (3b) wurde der Reaktor in gefluktem Zustand von unten nach oben durchströmt.

In der Rieselbettfahrweise (3a) wurde eine Ausbeute von 87,1 % erzielt. Nach der Reindestillation betrug die Produktreinheit 99,8 %; Methyl-IPDA war nur in einer Menge um 200 ppm nachweisbar.

In dem Versuch nach der Blasensäulenfahrweise (3b) wurde eine Ausbeute von 85,5 % erreicht. Die Reindestillation ergab nur eine Produktreinheit von 99,5 %. Die Analyse ergab, daß noch 3000 ppm Methyl-IPDA im Reinprodukt enthalten waren. Das die IPDA-Produktqualität sehr negativ beeinflussende Nebenprodukt Methyl-IPDA ist destillativ praktisch nicht abtrennbar.

### Beispiele 4 bis 6

Die Versuche wurden in der gleichen Versuchsanlage und unter gleichen Reaktionsbedingungen, wie unter Beispiel 1 beschrieben, durchgeführt. Variiert wurde lediglich der Katalysator. Die Ergebnisse folgen aus der Tabelle.

| Beispiel | Katalysator | Ausbeute IPDA (%) |
|---|---|---|
| 4 | 5 % Ru/γ-Al₂O₃ | 82,1 |
| 5 | Ni | 55,3 |
| 6 | 2 % Pd/γ-Al₂O₃ | 4 |

Das Ru und Pd war jeweils auf γ-Al₂O₃ Strangpreßlinge als Träger aufgebracht. Die Beispiele 5 und 6 verdeutlichen, daß im Vergleich zum erfindungsgemäßen Verfahren mit Ni- und Pd-Katalysatoren keine befriedigenden Ausbeuten erhalten werden.

### Beispiel 7

8770 g der Reaktionslösung aus Beispiel 2 wurde durch fraktionierende Destillation von den Leichtsiedern Methanol, NH₃, H₂O und Bicyclus befreit. Es blieb eine Rohlösung von 1292 g. Von dieser Lösung wurden bei einer Sumpftemperatur von 147 °C und einem Druck von 20 mbar 1190 g IPDA abdestilliert. Dies entspricht einer Isolationsausbeute von 87,3 %. Im Sumpf der Destillation verblieben 98 g. Aus diesem Sumpf wurde bei vermindertem Druck (10 mbar) und einer Temperatur von 162 °C eine weitere Hochsieder-Fraktion von 74 g abdestilliert. Es waren 20 % IPDA und 74 % Amidin enthalten. Diese erhaltene Fraktion von 74 g entspricht 5,6 % der eingesetzten IPN-Menge.

In einem weiteren Versuch, der analog Beispiel 2 durchgeführt wurde, wurde die Hochsiederfraktion, 5,6 % bezogen auf die eingesetzte IPN-Menge, zugesetzt. Nach der Hydrierung und Abtrennung der Leichtsieder und IPDA-Reindestillation ergab sich eine Isolationsausbeute von 92,3 %. Durch die Recyclierung der das Amidin enthaltenden Hochsiederfraktion konnte demnach eine IPDA-Isolationsausbeuteerhöhung um 5 % erzielt werden.

Durch eine katalytische Hydrierung der den Bicyclus enthaltenden Fraktion (Siedepunkt unterhalb IPDA) unter erhöhten Temperaturbedingungen in einem separaten Hydrierreaktor ist es möglich, die Ausbeute an IPDA um weitere 4 % zu erhöhen.

### Beispiele 8 bis 11

Es wurde die gleiche Versuchsanlage wie in Versuch 1 verwendet. Der Hydrierdruck betrug 60 bar, die Reaktionstemperatur 120 °C. Aus dem Abscheidegefäß wurde kein H₂ ausgeschleust. In der Einsatzlösung waren 30 Gew.-% IPN und 70 Gew.-% Methanol enthalten. Davon wurden 130 ml/h und zusätzlich 50 ml/h flüssiges NH₃ gemischt und in den Reaktor gepumpt. Diese Versuchseinstellung wurde mit einem Ruthenium-, einem Kobaltkatalysator und verschiedenen Kombinationen von beiden durchgeführt. Die Ergebnisse dieses Versuchs sind in der Tabelle dargestellt.

| Beispiel | Katalysator *) | Ausbeute (%) | Isomerenverhältnis cis : trans |
|---|---|---|---|
| 8 | 200 ml Co | 87,2 | 60 : 40 |
| 9 | 200 ml Ru | 81,5 | 84 : 16 |
| 10 | 100 ml Ru + 100 ml Co | 83,7 | 81 : 19 |
| 11 | 10 ml Ru + 190 ml Co | 86,9 | 78 : 22 |

| | | | |
|---|---|---|---|
| *) Ru-Katalysator: 5 % Ru auf γ-Al₂O₃-Strangpreßlingen Co-Katalysator: 50 % Co auf Al-silikat-Strangpreßling | | | |

Durch die Kombination eines Ru- und eines Co-Katalysators, wobei der Ru-Katalysator als obere Schicht im Reaktor angeordnet ist, gelingt es, IPDA mit einem hohen cis-/trans-Isomerenverhältnis in höherer Ausbeute zu erhalten als bei alleiniger Verwendung eines Ru-Katalysators.

### Beispiel 12

Ein Reaktionsrohr (Durchmesser 16 mm) einer Rieselbett-Hydrierapparatur wurde mit 120 ml handelsüblichem Co-Trägerkatalysator (Co auf Aluminiumsilikat, 50 Gew.-% Co; BET(N₂)-Oberfläche ca. 200 m²/g) gefüllt. Die Durchführung des Versuchs erfolgte analog Beispiel 1. Die Reaktionstemperatur wurde auf 110 °C einreguliert, der Gesamtdruck (Summe aus den Partialdrücken für H₂, NH₃ und Methanol) auf 60 bar. Die Einsatzlösung enthielt 15 Gew.-% Isophoronnitril, 65 Gew.-% Methanol und 20 Gew.-% Ammoniak. 100 ml dieser Einsatzlösung wurden pro Stunde auf das Rieselbett aufgegeben. Nach der Passage der Lösung durch das Rieselbett enthielt das Reaktionsgemisch (Mol-%, bezogen auf Isophoronnitril in der Einsatzlösung):

| | |
|---|---|
| Isophorondiamin (IPDA) | 89,2 % |
| Isophoronaminoalkohol (IPAA) | 2,1 % |
| Amidin (siehe Beispiel 1) | 4,0 % |
| Bicyclus (siehe Beispiel 1) | 3,8 % |

### Beispiel 13

Beispiel 12 wurde mit dem einzigen Unterschied wiederholt, daß ein anderer handelsüblicher Co-Trägerkatalysator verwendet wurde - Co auf Kieselsäure, 25 Gew.-% Co, BET(N₂)-Oberfläche ca. 200 m²/g. Das Reaktionsgemisch enthielt (Angabe in Mol-%, bezogen auf eingesetztes Isophoronnitril):

| | |
|---|---|
| IPDA | 87,4 % |
| IPAA | 1,2 % |
| Amidin | 4,3 % |
| Bicyclus | 5,1 % |

## Patentansprüche

1. Verfahren zur Herstellung von 3-Aminomethyl-3,5,5-trimethylcyclohexylamin (Isophorondiamin, IPDA) aus 3-Cyano-3,5,5-trimethylcyclohexanon (Isophoronnitril, IPN), indem ein Gemisch aus Isophoronnitril, einem organischen Lösungsmittel aus der Reihe der C₁- bis C₃-Alkohole und Ammoniak bei einem Druck im Bereich von 3 bis 10 MPa und einer Temperatur im Bereich von 80 bis 150 °C in Gegenwart mindestens eines Hydrierkatalysators aus der Reihe der Kobalt- und/oder Ruthenium-Festbettkatalysatoren mit Wasserstoff hydriert und das Reaktionsgemisch destillativ aufgearbeitet wird,
dadurch gekennzeichnet,
daß man die aminierende Hydrierung in einem Rieselbettreaktor durchführt, wobei das Gemisch aus Isophoronnitril, organischem Lösungsmittel und Ammoniak am oberen Ende des Reaktors auf das Katalysatorbett aufgegeben und das nach Durchrieseln des Katalysatorbetts erhaltene Reaktionsgemisch am unteren Ende des Reaktors abgenommen wird.

2. Verfahren zur Herstellung von Isophorondiamin nach Anspruch 1,
dadurch gekennzeichnet,
daß man die Hydrierung bei einer Temperatur im Bereich von 90 bis 130 °C und einem Druck von 5 bis 8 MPa durchführt.

3. Verfahren nach Anspruch 1 oder 2,
dadurch gekennzeichnet,
daß man als Lösungsmittel Methanol verwendet, das auf das Katalysatorbett aufgegebene Gemisch 10 bis 40 Gew.-%, vorzugsweise 10 bis 30 Gew.-%, Isophoronnitril und 10 bis 40 Gew.-%, vorzugsweise 20 bis 40 Gew.-%, Ammoniak enthält und das Gemisch eine homogene Lösung bildet.

4. Verfahren nach einem oder mehreren der Ansprüche 1 bis 3,
dadurch gekennzeichnet,
daß man als Hydrierkatalysator einen Trägerkatalysator verwendet, der im wesentlichen aus einem anorganischen Trägermaterial, vorzugsweise einem oxidischen oder silikatischen Trägermaterial, und Kobalt besteht, wobei der Katalysator insgesamt 10 bis 70 Gew.-% Kobalt enthält.

5. Verfahren nach einem oder mehreren der Ansprüche 1 bis 4,
dadurch gekennzeichnet,
daß man eine bei der destillativen Aufarbeitung des Reaktionsgemischs nach der Isophorondiamin-Hauptfraktion destillierende Hochsiederfraktion, enthaltend neben Resten an Isophorondiamin als Hauptkomponente 3,3,5-Trimethyl-6-imino-7-aza-bicyclo-[3,2,1]-octan, zusammen mit dem Gemisch aus Isophoronnitril, Lösungsmittel und Ammoniak dem Rieselbettreaktor zuführt.

6. Verfahren nach einem oder mehreren der Ansprüche 1 bis 5,
dadurch gekennzeichnet,
daß man die aminierende Hydrierung in einem Rieselbettreaktor mit einer darin angeordneten oberen Schicht aus einem Ruthenium-Trägerkatalysator, vorzugsweise Ruthenium auf γ-Aluminiumoxid, und einer unteren Schicht aus einem Kobalt-Festbettkatalysator durchführt, wobei die genannte obere Schicht und die genannte untere Schicht anstelle in einem Reaktor auch in zwei getrennten, in der genannten Reihenfolge hintereinander geschalteten Reaktoren angeordnet sein können.

7. Verfahren nach Anspruch 6,
dadurch gekennzeichnet,
daß das Volumen des Ruthenium enthaltenden Festbettkatalysators 3 bis 30 %, vorzugsweise 5 bis 20 %, des Gesamtvolumens an Festbettkatalysatoren ausmacht.

8. Verfahren nach einem oder mehreren der Ansprüche 1 bis 7,
dadurch gekennzeichnet,
daß man am oberen oder unteren Ende des Rieselbettreaktors Wasserstoff in einer solchen Menge zuführt, daß kein Wasserstoff aus dem Reaktor ausgetragen und rezykliert werden muß.

9. Verfahren nach einem oder mehreren der Ansprüche 1 bis 8,
dadurch gekennzeichnet,
daß man das Gemisch aus Isophoronnitril, Lösungsmittel und Ammoniak, das vorzugsweise 10 bis 30 Gew.-% Isophoronnitril und 20 bis 40 Gew.-% Ammoniak und Methanol als Lösungsmittel enthält, mit einem LHSV-Wert (liquid hourly space velocity) von 0,8 bis 1,5 h⁻¹ über das Katalysatorbett des Reaktors rieseln läßt.

## Claims

1. A process for the preparation of 3-aminomethyl-3,5,5-trimethylcyclohexylamine (isophoronediamine, IPDA) from 3-cyano-3,5,5-trimethylcyclohexanone (isophorone nitrile, IPN), wherein a mixture of isophorone nitrile, an organic solvent selected from the C₁- to C₃- alcohols and ammonia, at a pressure in the range from 3 to 10 MPa and at a temperature in the range from 80 to 150°C, is hydrogenated by hydrogen in the presence of at least one hydrogenation catalyst selected from the cobalt and/or ruthenium fixed bed catalysts and the reaction mixture is worked up by distillation, characterised in that the aminating hydrogenation is carried out in a trickle-bed reactor, the mixture of isophorone nitrile, organic solvent and ammonia being introduced at the upper end of the reactor onto the catalyst bed and the reaction mixture obtained after the reactants have trickled through the catalyst bed being withdrawn at the lower end of the reactor.

2. The process for the preparation of isophorone diamine according to claim 1, characterised in that the hydrogenation is carried out at a temperature in the range of from 90 to 130°C and at a pressure of from 5 to 8 MPa.

3. The process according to claim 1 or 2, characterised in that the solvent used is methanol, the mixture introduced onto the catalyst bed contains from 10 to 40 wt.%, preferably 10 to 30 wt.%, of isophorone nitrile and from 10 to 40 wt.%, preferably 20 to 40 wt.%, of ammonia and the mixture forms a homogeneous solution.

4. The process according to one or more of claims 1 to 3, characterised in that the hydrogenation catalyst used is a supported catalyst which consists substantially of an inorganic support, preferably an oxide or silicate material, and cobalt, the catalyst containing in total from 10 to 70 wt.% of cobalt.

5. The process according to one or more of claims 1 to 4, characterised in that a high-boiling fraction which distils off after the isophoronediamine main fraction during the working up of the reaction mixture by distillation and which contains, in addition to residues of isophoronediamine, 3,3,5-trimethyl-6-imino-7-azabicyclo-[3,2,1]octane as main component together with the mixture of isophorone nitrile, solvent and ammonia, is passed to the trickle-bed reactor.

6. The process according to one or more of claims 1 to 5, characterised in that the aminating hydrogenation is carried out in a trickle-bed reactor having an upper layer arranged therein consisting of a ruthenium supported catalyst, preferably ruthenium on γ-aluminium oxide, and a lower layer consisting of a cobalt fixed bed catalyst, the said upper layer and the said lower layer possibly also being arranged, instead of in one reactor, in two separate reactors arranged one behind the another in the sequence referred to.

7. The process according to claim 6, characterised in that the volume of the fixed bed catalyst containing ruthenium constitutes 3 to 30%, preferably 5 to 20%, of the total volume of fixed bed catalyst.

8. The process according to one or more of claims 1 to 7, characterised in that hydrogen is supplied at the upper or lower end of the trickle-bed reactor in a quantity such that no hydrogen is discharged from the reactor and has to be recirculated.

9. The process according to one or more of claims 1 to 8, characterised in that the mixture of isophorone nitrile, solvent and ammonia, which preferably contains from 10 to 30 wt.% of isophorone nitrile and from 20 to 40 wt.% of ammonia and methanol as solvent, is caused to trickle over the catalyst bed of the reactor at an LHSV value (liquid hourly space velocity) of from 0.8 to 1.5 h⁻¹.

## Revendications

1. Procédé d'obtention de la 3-aminométhyl-3,5,5-triméthylcyclohexylamine(isophoronediamine IPDA) à partir de 3-cyano-3,5,5-triméthylcyclohexanone(isophoronenitrile, IPN) dans lequel on hydrogène un mélange d'isophoronenitrile, d'un solvant organique choisi de la série des alcools en C₁ à C₃ et d'ammoniac à une pression dans la zone de 3 à 10 MPa et à une température dans la plage de 80 à 150°C, en présence d'au moins un catalyseur d'hydrogénation choisi dans la série des catalyseurs en lit solide au cobalt et/ou au ruthénium, avec de l'hydrogène et dans lequel on traite par distillation le mélange réactionnel,
caractérisé en ce que
l'on effectue l'hydrogénation aminante dans un réacteur à lit de ruissellement dans lequel on verse le mélange d'isophoronenitrile, de solvant organique et d'ammoniac à l'extrémité supérieure du réacteur sur le lit de catalyseur et on retire le mélange réactionnel obtenu après traversée par ruissellement du lit de catalyseur, à l'extrémité inférieure du réacteur.

2. Procédé d'obtention d'isophoronediamine selon la revendication 1,
caractérisé en ce que
l'on effectue l'hydrogénation à une température dans la plage de 90 à 130°C et à une pression de 5 à 8 MPa.

3. Procédé selon la revendication 1 ou la revendication 2,
caractérisé en ce que
l'on utilise comme solvant le méthanol, le mélange versé sur le lit de catalyseur contient de 10 à 40 % en poids, de préférence de 10 à 30 % en poids, d'isophoronenitrile et de 10 à 40 % en poids, de préférence de 20 à 40 % en poids d'ammoniac et le mélange forme une solution homogène.

4. Procédé selon l'une ou plusieurs des revendications 1 à 3,
caractérisé en ce que
l'on utilise comme catalyseur d'hydrogénation un catalyseur sur support qui est composé essentiellement d'un matériau de support minéral, de préférence un matériau de support par voie d'oxydation ou silicaté, et de cobalt, le catalyseur au total renfermant de 10 à 70 % en poids de cobalt.

5. Procédé selon l'une ou plusieurs des revendications 1 à 4,
caractérisé en ce que
l'on amène au réacteur à lit de ruissellement une fraction de produits à point d'ébullition élevé distillant après la fraction principale - l'isophoronediamine - au cours du traitement par distillation du mélange réactionnel, qui contient à côté des résidus d'isophoronediamine comme composant principal, du 3,3,5-triméthyl-6-imino-7-azabicyclo[3-2-1}-octane conjointement au mélange d'isophoronenitrile, de solvant et d'ammoniac.

6. Procédé selon l'une ou plusieurs des revendications 1 à 5,
caractérisé en ce que
l'on effectue l'hydrogénation aminante dans un réacteur à lit de ruissellement avec une couche supérieure disposée dans celui-ci d'un catalyseur sur support au ruthénium, de préférence du ruthénium sur de l'oxyde d'aluminium-γ et avec une couche inférieure d'un catalyseur sur lit solide au cobalt, la couche supérieure citée et la couche inférieure citée pouvant être disposées en utilisant à la place d'un seul réacteur, deux réacteurs séparés, connectés l'un derrière l'autre dans la séquence citée.

7. Procédé selon la revendication 6,
caractérisé en ce que
le volume du catalyseur en lit solide contenant le ruthénium représente de 3 à 30 %, de préférence de 5 à 20 % du volume total de catalyseur en lit solide.

8. Procédé selon l'une ou plusieurs des revendications 1 à 7,
caractérisé en ce que
l'on amène à l'extrémité supérieure ou inférieure du réacteur en lit de ruissellement de l'hydrogène en quantité telle, qu'on n'évacue pas d'hydrogène du réacteur et qu'on ne doit pas le recycler.

9. Procédé selon l'une ou plusieurs des revendications 1 à 8,
caractérisé en ce que
l'on peut faire ruisseler le mélange d'isophoronenitrile, de solvant et d'ammoniac, qui renferme de préférence de 10 à 30 % en poids d'isophoronenitrile et de 20 à 40 % en poids d'ammoniac et de méthanol comme solvant ayant une valeur LHSV (liquid hourly space velocity) de 0,8 à 1,5 h⁻¹ sur le lit de catalyseur du réacteur.
